# EUROPEAN PATENT SPECIFICATION

(11) **EP 4 258 711 B1**
(45) Date of publication and mention of the grant of the patent: **15.01.2025**
(21) Application number: 22382333.7
(22) Date of filing: 06.04.2022
(51) Int. Cl.: H04W 4/80, A61B 5/00

(54) **METHOD AND SYSTEM FOR REMOTE MONITORING OF A VITAL SIGN OF A PATIENT**
VERFAHREN UND SYSTEM ZUR FERNÜBERWACHUNG EINES VITALZEICHENS EINES PATIENTEN
PROCÉDÉ ET SYSTÈME DE SURVEILLANCE À DISTANCE D'UN SIGNE VITAL D'UN PATIENT

(43) Date of publication of application: 11.10.2023
(73) Proprietor: Vitio Medical, S.L., 31192 Aranguren (ES)
(72) Inventor: RUIZ TADEO, Fernando José, 31192 TAJONAR (ES); ELCOSO USIETO, Ignacio, 31192 TAJONAR (ES); INDURAIN HUARTE, Mikel, 31192 TAJONAR (ES); SESMA SÁNCHEZ, Laura, 31192 TAJONAR (ES); GOYENA GARCÍA-TUÑÓN, Miguel, 31192 TAJONAR (ES); RUBÉN ANAUT DOMEÑO, 31192 TAJONAR (ES)
(74) Representative: ZBM Patents - Zea, Barlocci & Markvardsen

(56) References cited:
- US-A1- 2019 116 088
- US-A1- 2020 121 937
- US-A1- 2021 020 309

## Description

### BACKGROUND

Bluetooth Low Energy (BLE) technology is widely used to determine the physical location of devices, to track objects or to trigger location-based actions at certain points of interest, such as in a bus stop, a store, or a location inside a building. It is also used in retail, where beacons deliver content based on proximity (for example for geo-targeted marketing). These systems use BLE advertising mode, sending their advertising URLs via, for example, an Eddystone-URL frame. However, when a system wants to send sensor data to a receiver, embedded in a computing device, both industrial and medical devices normally use advertising mode to initiate a connection with another computing device (for example, a computing server or a portable device such as a smartphone), and switch to connection mode to send the bulk of the data obtained by the sensor.

In a health monitoring environment, such as a hospital or a nursing home, a monitoring of vital sign data from the patients may be performed by means of a vital sign sensor attached to each patient. Furthermore, there may be one or more receiving computing devices distributed within the hospital in such a way that they cover the whole building, enabling a BLE communication between the receivers and the sensors. When using a BLE communication in connection mode to send the data obtained by the sensors to one or more receivers, there is a limitation in the number of sensors that can be connected simultaneously to each receiver, requiring at least one receiver per 8 sensors.

However, it is also known to send data captured from a sending device (such as sensor devices) to a computing device within a BLE advertising type frame, wherein there is no limitation of number of sensors per receiving computing device.

When using a BLE communication, either sending data in connection mode or within advertising type frames, important parts of data obtained and sent by a sensor may not always reach a receiving computing device. For example, when monitoring the health of a patient at home during a home hospitalization, a receiver may be located in a central part of the house, and it may receive data from a sensor attached to the patient, wherein the sensor may not always be within reach of the receiver. For example, when the patient walks far away from that part of the house (the patient goes for a walk), the signal from the sensor may be lost by the receiver.

Furthermore, a patient may be in a place but move its resting position (for example, when sleeping), obstructing the correct sending of the data from the sensor to a receiver. Such loss of data may happen in an unpredictable way (depending on, for example, if a patient hospitalized at home moves far away from the room where the receiver is located) or in a periodical and predictable way (for example, when the patients sleep, the sensor may usually have more obstructions to correctly send the sensor data to the receiver).

Therefore, a system for monitoring a vital sign of a patient may be needed, which guarantees a low loss of data, thus ensuring a continuous and correct monitoring of vital signs of a patient.

Prior art document US2019/116088 A1 discloses a solution with switching from advertising to connection mode for sending patient sensor data. However, the criteria to switch is only about the content of the monitored data reaching critical values.

### SUMMARY

According to a first aspect, a method of remote monitoring of a vital sign of a patient is provided. More precisely, the method is performed by a computing device comprising a BLE communication module to communicate with a vital sign sensor, the method comprising the steps of:
- Receiving a first BLE advertising communication packet comprising a vital sign data packet, from the sensor;
- Generating a timestamp for the received first BLE advertising communication packet;
- Performing a first time verification of if the difference between the generated timestamp and a stored timestamp of a previously received BLE advertising communication packet is higher than a predetermined waiting time:
   ∘ In case of positive result, sending a BLE packet from the computing device to the vital sign sensor, to establish a BLE communication in connected mode between the vital sign sensor and the computing device, and retrieving, using the BLE communication in connected mode, a plurality of vital sign data packets from the sensor, wherein the packets correspond to timestamps with values between the generated timestamp and the stored timestamp.

The method of remote monitoring is performed by a computing device which can be a smartphone, a tablet, a computer, or any similar device that comprises a BLE communication module, thus being able to communicate with other devices using a BLE protocol. Furthermore, the computing device is able to connect to a vital sign sensor using said BLE communication module, embedding data using a BLE protocol. More precisely, the vital sign sensor may be a sensor for capturing data related to, for example, body temperature, blood pressure, respiration and heart rates, oxygen saturation, electrocardiogram, blood glucose concentration, or brain waves from a patient.

According to the above method, the computer device receives a first BLE advertising communication packet from the vital sign sensor.

Bluetooth Low Energy (otherwise known as BLE) is a subset of the Bluetooth standard. It has a unique protocol stack in reference to the OSI layer and is oriented to simple connections in very low power applications useful for (battery-dependent devices).

As a communications protocol, BLE has very low power requirements (consumes 5% to 90% less energy than standard Bluetooth), has a much lower cost and an acceptable range in communications. Because of this, many smart home, health, automotive and even security devices use this type of communication protocol between the devices and the applications which control them.

Furthermore, any BLE protocol comprises at least two modes of communication: advertising mode and connected mode. Advertising mode communications are defined by the Generic Access Profile (GAP), wherein such communications are used to transmit data to any device who may be listening. On the other hand, connected mode communications are defined by the Generic Attribute (GATT), wherein such communications are used to transfer data through an already established Bluetooth connection between two devices.

Therefore, advertising mode may be defined as a one-to-many type of communication (i.e., a broadcast type of communication), while connected mode may be defined as a communication between two devices (i.e., a master - slave type of communication).

Advertising mode uses BLE advertising communication data packets to embed data, which may be defined by different types of advertising protocols within BLE advertising mode. The BLE advertising protocols define frames which comprise, generally, a header comprising data about the version of the protocol, length of the frame and command identifiers, and a payload section comprising useful information of the protocol.

The main existing BLE advertising protocols are, among others:
- iBeacon: created by Apple, which was first introduced at the Worldwide Developers Conference 2013. Apple was the first company to make this technology known worldwide, but the technology (BLE) was created by Nokia.
- Eddystone: Google's open-source project for beacons. With this technology, Google aims to promote the internet of things. Similar to iBeacon protocol, but open source. However, iBeacon is officially supported by iOS devices only, and Eddystone has official support for iOS and Android.

Both iBeacon and Eddystone (or other types of BLE advertising protocols) embed data in a frame, wherein the frame comprises, as previously mentioned above, a plurality of fields to control and manage the communications between devices (within the header section), and a plurality of optional fields (within the payload section), which can be used by the device to send specific information about the state of the sending device, such as telemetry data related to the strength of the signal.

On the other hand, in connected mode, GATT (Generic Attribute Profile) defines how two BLE devices can communicate using the Services and Features. Once a dedicated connection has been established between two devices, the communication in connected mode is defined by GATT. In order to establish such communication in connected mode, a communication through the advertising mode (defined by GAP) has previously been carried out.

The most important characteristic of communications in connected mode is that they are exclusive. A BLE peripheral (such as a vital sign sensor) can only be connected to one central device (a computing device such as, for example, a smartphone) at a time. As soon as a peripheral is connected to a central device, it will stop advertising itself and other devices will no longer be able to see it or connect to it, until the existing connection is terminated.

When using BLE, establishing a communication in connection mode enables bidirectional communication, wherein the computing device can send data to the peripheral (for example, a vital sign sensor), and the peripheral can respond (and vice versa).

According to the method of the present disclosure, the received BLE advertising communication packet comprises a vital sign data packet. Such packet comprises data corresponding to at least one measurement obtained by the vital sign sensor. For example, the vital sign data packet may comprise data corresponding to a plurality of body temperature measurements performed by a body temperature sensor. Furthermore, for each portion of data corresponding to a sensor measurement, a timestamp is further generated. Said timestamp may be generated locally within the computing device (upon the arrival of the BLE advertising communication packet) or it may be obtained from the BLE advertising communication packet itself (for example, the timestamp may have been generated within the sensor, and embedded within the packet before being sent to the computing device). This way, the computing device receives data corresponding to the value of at least one measurement performed by the sensor, and when that measurement was performed (i.e., the generated timestamp, whether generated at the computing device or obtained from the received BLE advertising communication packet). Furthermore, the vital sign data packet may be embedded within one or more of the previously described optional fields of the BLE advertising frame. This way, by complying with the BLE protocol, the inclusion of a vital sign data packet does not affect the management and control of communications between the vital sign sensor and the computing device.

Afterwards, a verification is performed to determine if the difference between the generated timestamp and a stored timestamp of a previously received BLE advertising communication packet is higher than a predetermined waiting time. Thus, said verification is performed to assess if more than a certain time has passed between the reception of two BLE advertising communication packets.

Furthermore, for example, the previously described verification related to the difference between the generated timestamp and a stored timestamp would be useful if the user wearing the sensor has moved out of reach of the computing device. More precisely, if the computing device is a smartphone, the user may have left the smartphone in a room and may have walked out of the building. In this case, the computing device may have not received vital sign data embedded within one or more BLE advertising communication packets sent by the sensor while the computing device was out of reach of the sensor, thus losing said vital sign data, generated while being out of reach.

Subsequently, in case of positive result of the verification (i.e., a certain waiting time between the reception of two packets has passed), a BLE packet is sent from the computing device to the vital sign sensor, to establish a BLE communication in connected mode between the vital sign sensor and the computing device. Such BLE packet may be a request using any BLE protocol to establish a master-slave type of communication (i.e., a connected mode type of communication) between the computing device and the vital sign sensor. However, a specific set of instructions may be defined within the BLE protocol in order to perform the retrieval of vital sign data packets from the sensor, and, optionally, any timestamp corresponding to such packets.

Afterwards, through the established BLE communication in connected mode, a plurality of vital sign data packets are retrieved from the vital sign sensor (for example, from a memory connected to the sensor) and sent to the computing device. Such packets may be stored within a memory connected to the sensor and may correspond to timestamps with values between the received timestamp and the stored timestamp.

The predetermined waiting time may be determined by, for example, the specific application of the system. That is: if the aim of using the computing device and the sensor is to monitor the body temperature of a patient, the waiting time may be higher, since body temperature variations are slower than variations of other vital signs. Similarly, the monitoring of a patient's body temperature may be more critical when the patient has fever (more than 37°C), thus choosing a shorter waiting time during fever episodes. Another example may be when the aim is to monitor blood pressure or heart rate of a user, wherein the monitoring is more reliable when the user is resting, and specially when he/she is sleeping. Therefore, in summary, the waiting time may be adjusted depending on the state of the patient, the type of monitoring being performed, or the time schedule of, for example, a doctor in charge of the monitoring.

In many cases, BLE advertising communication packets are used to initiate a communication in between two devices, which once initiated, is switched to a dedicated communication in between them in connected mode. Furthermore, such BLE advertising communication packets are also used to transmit telemetry data within the optional fields of its frame, which is useful for monitoring the performance of beacons or devices (i.e., elements of the devices which send such packets). Such telemetry is used, for example, by other receiving devices (any device reached by the BLE advertising packet) to assess if it is possible to establish a further BLE communication in connected mode.

However, the method of the present disclosure uses the optional data fields (which can comprise, for example, telemetry data) of BLE advertising frames to send a vital sign data packet, thus being able to send data related to vital sign measurements (such as body temperature, blood pressure, etc...) obtained from a vital sign sensor connected to the computing device.

Therefore, a BLE advertising communication packet (i.e., a packet sent using a BLE advertising mode protocol) is used to transmit vital sign data, instead of sending such vital sign data packets using a BLE connection mode type of communication (using BLE or other standard Bluetooth connection protocols in connected mode). This way, generally, by using such advertising packets instead of a connection mode type of communication, a substantial saving of energy is achieved within the vital sign sensor (that is, a saving of energy of the batteries of the vital sign sensor).

Furthermore, by controlling the periods when the computing device may periodically be within reach of the sensor, using a verification and a subsequent obtaining of vital sign data packets sent by the sensor while it was out of reach of the computing device, the frequency of the broadcasting (i.e., sending) of BLE advertisings packets may be adjusted for extra energy saving. For example, in a case when a patient wearing a sensor of the system uses the bathroom on a regular basis, or is out of reach of the computing device at predictable times during the day (for example, at lunch time, or during the night, while sleeping, when the sensor cannot reach the computing device properly), an extra saving can be achieved by avoiding the sending of BLE advertising mode packets during such periods of time, and waiting for the period of time for the computing device to most likely be reachable by the sensor.

Thus, according to another example, the method of remote monitoring of a vital sign of a patient further comprises a step of determining a pattern of availability of the sensor, based on at least one difference between a generated timestamp and a stored timestamp.

Because of this, the autonomy of the vital sign sensor (i.e., the autonomy of the battery of the vital sign sensor) can be substantially increased by adjusting the sending of the BLE advertising packets by the sensor, based on previous (single or multiple) iterations of the method, wherein it is possible to identify the patterns of availability of the sensor.

In addition to this, the vital sign data may not be lost while the sensor is out of reach of the computing device, and a single connection mode type of communication between the sensor and the computing device may be used in between periods where both devices are within reach and out of reach, to retrieve any lost data.

This is particularly useful when, for example, a substantially long period of time has passed between the BLE advertising communication packet last received by the computing device (for example, a packet that was stored by the computing device when received) and a new arriving BLE advertising communication packet.

Furthermore, a longer working distance may be achieved at a low computational cost. More precisely, there are receivers (for example, smartphones) that restrict the minimum power needed to maintain a BLE communication on a connected mode with a sensor (thus affecting the distance at which a connected mode type of communication can be established and/or maintained). This restriction is performed in order to avoid saturating the use of the antenna of the computing device (for example, a smartphone). This is because when both a sensor and a computing device are found nearby, within a limit distance between them (a zone wherein signal is weak) and the power of the BLE communication receiving signal is subsequently reduced, the receiving device uses a substantial amount of energy to ensure a stable connection (for example, by performing scans of the sensor, or restarting the connection between them). Therefore, some computing devices prevent the saturation of the antenna by limiting the required energy of the received signal to a minimum, to establish a communication in connected mode. Furthermore, there is no limitation of number of sensors per receiving device. When using a BLE communication in connection mode to send the data obtained by the sensors to one or more receivers, there is a limitation in the number of sensors that can be connected simultaneously to each receiver, requiring at least one receiver per 8 sensors. Therefore, by using BLE advertising mode type of communications, such limitations may be avoided.

According to an example of the present disclosure, the vital sign data packet may comprise data corresponding to at least one body temperature measurement performed by the vital sign sensor, and the method may further comprise a step of:
- verifying if the body temperature measurement is higher than a predetermined temperature threshold;
and wherein, in case of positive result of the temperature verification, the value of predetermined waiting time is lowered.

According to an example of the present disclosure, the method of remote monitoring of a vital sign of a patient may further comprise the step of:
- verifying if the received signal comprising the first BLE advertising communication packet has a higher power than a predetermined power threshold;
and wherein the step of retrieving a plurality of vital sign data packets from the sensor is performed only in case of positive result of the first power verification.

More specifically, the power or RSSI (Received Signal Strength Indicator) of the received signal comprising the first BLE advertising packet may be measured, and compared to a predetermined power threshold. Said threshold may be a predetermined minimum power in order to establish a connected mode communication between the sensor and the computing device, which is most likely to be maintained while the subsequent retrieval of the packets is performed. Such situation may be important when a sensor is within the limit of the range of its advertising signal with the computing device (i.e., the computing device is almost out of reach of the sensor, so a connected mode type of communication may easily be interrupted if the sensor or the computing device move slightly further away from each other). Once a connected mode communication is established, and a positive result is obtained from said first power verification, the retrieval is performed. However, if a negative result is obtained from said first power verification, it would mean that, if the connected mode communication was established, it was likely to be interrupted while the retrieval was being performed. This would result in, for example, having to start the retrieval from the beginning, thus losing time and energy by doing so.

The predetermined minimum power limitation previously described may be established by each Operative System, by means of, for example, specific libraries or its preprogramed functions. Furthermore, an additional limitation may be established when performing the method according to the present disclosure, in order to further save battery while establishing a connected mode type of communication.

According to another example, the method of remote monitoring of a vital sign of a patient may further comprise the steps of:
- receiving a second BLE advertising communication packet after the first received packet; and
- verifying if the received signal comprising the second BLE advertising communication packet has a higher power than a predetermined power threshold;
and wherein the step of retrieving a plurality of vital sign data packets from the sensor is performed only in case of positive result of the first power verification and the second power verification.

More specifically, to ensure that the retrieval of vital sign data packets using a connected mode communication will be less likely to be interrupted, a second power verification may also be performed (besides the first one corresponding to the first received BLE packet) on the signal comprising the second BLE advertising packet. This way, it may be verified that at least two received signals comprising BLE advertising packets are strong enough to further perform the subsequent retrieval (i.e., the signal coming from the sensor is strong enough to maintain a connected mode communication between the sensor and the computing device after being established).

According to another example, the method of remote monitoring of a vital sign of a patient further comprises the step of:
- Generating a second timestamp for the second BLE advertising communication packet;
- Performing a time verification of if the difference between the timestamp of the second BLE advertising communication packet and the timestamp of the first BLE advertising communication packet is lower than a predetermined waiting time;
and wherein the step of retrieving a plurality of vital sign data packets from the sensor is performed only in case of positive result of the first and second power verification and the second time verification.

Therefore, a further time verification may be performed, in order to determine if the difference between the timestamps of each first and second received BLE advertising packets (i.e., the time elapsed between receiving the first and the second BLE advertising packets) is lower than a predetermined waiting time. In case of positive result, the retrieval may be further performed. This may be useful when assessing that the receiving of two or more BLE advertising packets is close enough (in time), thus determining that the subsequent communication in connected mode may be reliable enough, thus ensuring that the subsequent retrieval may not be likely to be interrupted. Furthermore, as in the case of the received first BLE packet, a timestamp corresponding to the second BLE packet may be generated at the computing device (for example, upon arrival of the second BLE packet to the computing device), or it may be obtained from the second BLE packet (i.e., the timestamp being generated by the sensor at the time that the measurement or measurements corresponding to the vital sign data packet were performed).

According to an example, the received BLE advertising communication packet may be embedded in an Eddystone frame.

More specifically, Eddystone is a Google protocol specification that defines a Bluetooth Low Energy (BLE) message format for proximity beacon messages. It describes several different frame types that can be used individually or in combinations to create beacons that can be used for a variety of applications.

Eddystone frames carry sensor information (for sensing the temperature and/or humidity of the device itself), which makes it very useful for Internet of Things.

The common frame PDU (protocol data unit) types and individual service data byte arrangements for Eddystone frame formats are:
- Eddystone-UID: it serves as the identifier for Beacons as much as the combination of UUID, major and minor does in the iBeacon standard;
- Eddystone-URL: it broadcasts a URL using a compressed encoding format to fit more into the limited advertising packet. Once decoded, the URL can be used by any client with Internet access;
- Eddystone-TLM: Eddystone Beacons can transmit data about their own performance to clients. This data is called telemetry and is useful for monitoring the performance of a fleet of beacons (i.e., the devices which send the Eddystone frames). Since the Eddystone-TLM frame does not contain a Beacon ID that identifies the device, it may be paired with an ID frame that provides the ID, either of the Eddystone-UID or Eddystone-URL type in order to uniquely link telemetry to a device.

In Eddystone protocol, the "TLM" frame indicates that the frame is providing telemetry from a device. The data packet specification may be as follows:
- Battery voltage is in millivolts and defaults to zero if the device does not support it.
- Temperature is expressed in degrees Celsius using the fixed point notation with sign 8.8 and defaults to -128°C if the device does not support it. In the case of devices such as temperature sensing beacons, the TLM temperature is obtained from a dedicated temperature and humidity measurement sensor for greater accuracy and repeatability.
- ADV_CNT is the number of advertisement frames sent since device startup and SEC_CNT is the elapsed time since device power-up. These can be used to infer time and duration.

More precisely, according to an example, the two existing bytes found within an Eddystone TLM frame, designed to send the beacon temperature, may be used to send a vital sign data packet comprising data related to, for example, body temperature of the patient. Therefore, the data retrieved from the sensor is sent within an Eddystone TLM frame, thus maintaining device interoperability.

This way, by using the telemetry part of an Eddystone frame to send a vital sign packet, such as a measurement of the vital sign sensor (for example, temperature measurement from the sensor), the format of the frame remains the same, and a while a vital sign measurement can be extracted by any computing device receiving the BLE advertising communications packet. Furthermore, Eddystone remains compatible with any operating system, thus ensuring interoperability with any type of computing device (such as, for example, different types of smartphones using different operating systems). Also, Eddystone frames may be used for sending BLE advertising communications packets, while a dedicated protocol may be used to retrieve other packets from the vital sign sensor when a BLE connection in connected mode is established.

Alternatively, other BLE advertising communication packets could likewise be sent using iBeacon protocol.

According to another aspect of the present disclosure, a computing device for remote monitoring of a vital sign of a patient is presented, the computing device comprising:
- a BLE communication module to communicate with a vital sign sensor;
- a connection to a BLE advertising communication packets repository;
- a controller configured to:
   ∘ receive a first BLE advertising communication packet comprising a vital sign data packet, from the sensor;
   ∘ generate a timestamp for the received first BLE advertising communication packet;
   ∘ Perform a first time verification of if the difference between the generated timestamp and a stored timestamp of a previously received BLE advertising communication packet, stored in the repository, is higher than a predetermined waiting time, and:
      ▪ In case of positive result, send a BLE packet from the computing device to the vital sign sensor, to establish a BLE communication in connected mode between the vital sign sensor and the computing device, and retrieve, using the BLE communication in connected mode, a plurality of vital sign data packets from the sensor, wherein the packets correspond to timestamps with values between the generated timestamp and the stored timestamp.

The computing device may comprise a connection to a BLE advertising communication packets repository. Such repository may be, for example, found within a server in the cloud, or within the internal memory of the computing device (for example, within the internal memory of a smartphone). Furthermore, the repository may comprise at least one timestamp related to at least one BLE advertising communication packet previously received by the computing device.

Furthermore, according to another aspect of the present disclosure, a computer program is presented comprising program instructions for causing a computing device to perform the previously described method of remote monitoring of a vital sign of a patient. Also, in an example, the computer program may be embodied on a storage medium, or carried on a carrier signal.

### BRIEF DESCRIPTION OF THE DRAWINGS

Non-limiting examples of the present disclosure will be described in the following, with reference to the appended drawings, in which:
Figure 1 shows a system for monitoring a vital sign of a patient according to an example of the present disclosure;
Figure 2 shows a bracelet comprising a sensor, according to an example of the present disclosure;
Figure 3 shows a schematic representation of the steps of an example of the method of monitoring a vital sign of a patient according to an example of the present disclosure;
Figure 4 is a diagram representing a BLE packet configuration, according to an example of the present disclosure.

### DETAILED DESCRIPTION OF EXAMPLES

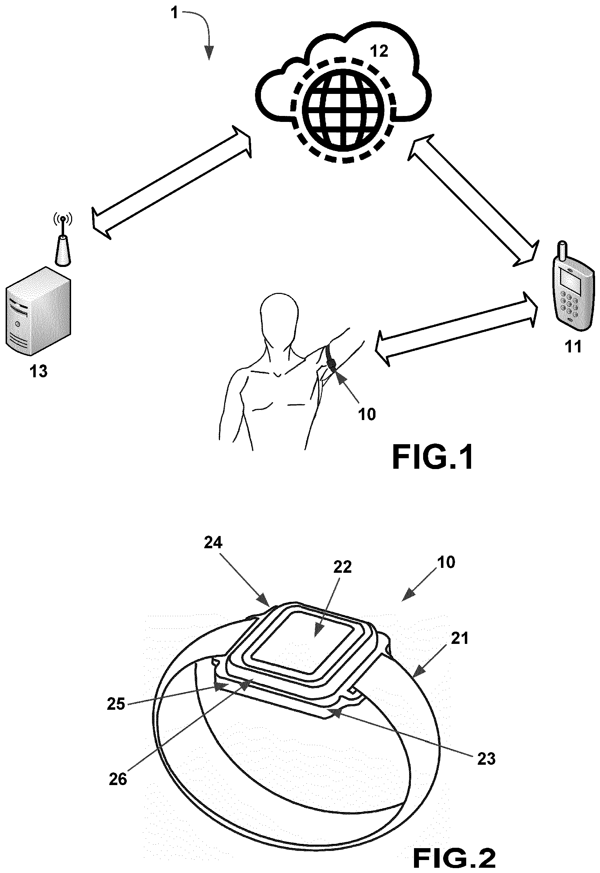

According to an example of the present disclosure, as seen in figure 1, a system 1 for monitoring the vital sign of a patient is presented. More precisely, the system 1 is configured to monitor the body temperature of a patient, and it comprises a vital sign sensor 10, and a computing device 11, which is a smartphone. Both the sensor 10 and the smartphone 11 comprise each a Bluetooth Low Energy (BLE) communications module, thus being able to communicate between each other using a BLE protocol.

In this example, as seen in figure 2, the vital sign sensor 10 is a body temperature sensor comprising a holding cuff 21, which can be placed and adjusted in the axillary area of the patient, and a sensing element device 22, which can be attached to the holding cuff in order to be placed in the axillary area, to perform a continuous measurement of the body temperature in the axillary area. Furthermore, the vital sign sensor 10 comprises a housing, and within the housing it further comprises a battery 23 to provide energy to the sensor, a controller 24 to manage the performance of the sensor 10, a BLE communications module 25, and an internal memory 26, connected to the controller, to store measurements performed by the sensing element device 22.

More specifically, in this example, the sensor 10 is provided with an internal memory 26 where it stores data related to temperature measurements obtained by the sensing element device 22, in a periodic continuous way. The data stored within the internal memory 26 is structured using a header section, a data section, and a tail section, wherein the header and the tail section provide the temperature data. They also allow to differentiate the data corresponding to different temperature measurements, in case there are more than one.

The header comprises several fields, which are mainly START, TIMESTAMP (UTC date and time at which the start or stop recording is made), GUID (a field to uniquely identify the recording), STOP (which allows to identify when a capture of the measurement ends), and TIME SINCE LAST CAPTURE (time elapsed between the last measurement and the capture stop), among others. Thus, each different temperature measurement has a corresponding timestamp, related to the time when the measurement was performed.

More precisely, in this example, each time the sensing element device 22 of the vital sign sensor 10 obtains data related to a temperature measurement of the patient, the data is stored within the internal memory 26 of the sensor 10, and also, by means of the controller 24 and the BLE communications module 25, said data is embedded within a BLE advertising communication packet and further broadcasted by the vital sign sensor 10.

In the present example, as seen in figure 1, the smartphone 11 is further connected to a cloud 12, via an Internet connection. Also, a server 13 is also connected to the cloud 12. The server 13 allows monitoring data obtained by the smartphone 11, or to manage the smartphone 11 remotely.

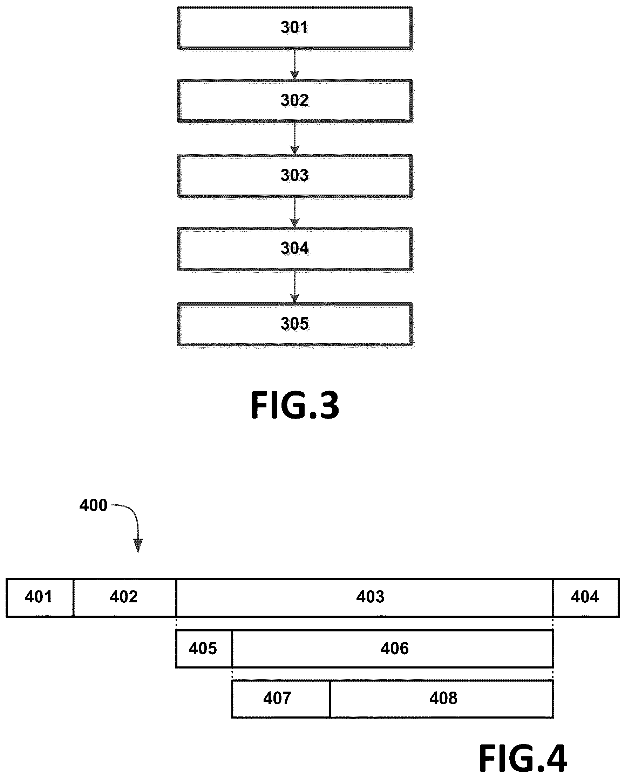

Furthermore, figure 3 depicts an example of a method of remote monitoring of a vital sign of a patient according to the present disclosure. More precisely, the method is performed by the smartphone 11, and it comprises the steps of:
- Step 301 of receiving a first BLE advertising communication packet comprising a vital sign data packet (comprising data related to the temperature of the patient) and a corresponding timestamp, from the vital sign sensor 10.

More precisely, the sensing element device 22 of the sensor 10 obtains a temperature measurement from the patient, and the controller 24 of the sensor 10 embeds the data related to the temperature measurement within a BLE Eddystone advertising communication packet and sends the packet using the BLE communications module 25 of the sensor 10. This way, when the patient is within reach of the smartphone, the smartphone receives the packet through such communication in advertising mode, sent by the sensor 10.

Afterwards, the following step is performed:
- Step 302 of verifying if the difference between the received timestamp and a stored timestamp of a previously received BLE advertising communication packet is higher than a predetermined waiting time.

In this case, the smartphone 11, in an internal memory (not pictured), has stored the last BLE advertising communication packet received before the actual packet, with its corresponding timestamp. Such timestamps (the one corresponding to the present packet and the one corresponding to the packet last received by the smartphone) are compared, and a difference of time between them is determined. Such difference of time is the amount of time elapsed between the last received packet by the smartphone 11 and the present packet received by the smartphone 11.

Then, a further step is performed depending on the resulting difference of time (or time elapsed):
- Step 303 of, in case of positive result (i.e., the difference of time is higher than a predetermined waiting time), a BLE packet is sent from the smartphone 11 to the vital sign sensor 10, to establish a BLE connection between the vital sign sensor 10 and the smartphone 11.

Such BLE packet is a request to the controller 24 of the sensor 10 to establish a subsequent BLE communication in connected mode between the smartphone 11 and the sensor 10. This may be performed by sending a specific BLE advertising mode request using a specifically designed BLE protocol, with instructions for the sensor 10. In response, the sensor 10, by means of its controller, may establish a BLE communication in connected mode between itself and the smartphone 11.

Furthermore, after establishing the BLE communication in connected mode between the smartphone 11 and the sensor 10, a further step is performed:
- Step 304 of retrieving, using the BLE communication in connected mode, a plurality of vital sign data packets from the sensor 10, wherein the packets correspond to timestamps with values between the received timestamp and the stored timestamp.

Such packets are retrieved from the internal memory 26 of the sensor 10, by indexing the packets of data corresponding to measurements obtained by the sensor in between the last received measurement stored within the smartphone 11, and the present measurement received by the smartphone 11. This way, all the data which has been continuously sent in BLE advertising communication packets by the sensor 10, but the smartphone 11 has not received due to, for example, being out of reach of the sensor 10, are sent through a BLE communication in connected mode from the sensor's 10 internal memory 26 (using the BLE communications module 25) to the smartphone 11.

Afterwards, the following step is performed:
- Step 305 of determining a pattern of availability of the sensor, based on the difference between the received timestamp from the first BLE advertising communication packet and a plurality of stored timestamps corresponding to a plurality of previously stored BLE advertising communication packets.

More precisely, the smartphone 11 may compare the timestamp of the present BLE advertising packet with a plurality of timestamps from previously received BLE advertising packets received by the smartphone. This way, the frequency of reception of the BLE advertising packets from a sensor may be determined, thus being also able to determine a pattern of availability of the sensor 10. For example, it may be determined that from 9pm to 8am, the patient sleeps and his/her body substantially blocks the signals sent by the sensor 10. This may be useful in order to save even more battery of the sensor, by establishing that the sensor may be saving the periodical temperature measurements within its internal memory 26, while not sending BLE advertising packets with data related to said temperature measurements. This way, the sensor can start sending current BLE advertising packets at the set time (from 8am onwards), so the smartphone 11 can perform the method of the present disclosure, and retrieve all the data related to measurements performed between 9pm and 8am, while energy has been saved during the night by the sensor 10 not sending any BLE advertising packet.

Furthermore, the smartphone 11 is also connected through the cloud 12, to a computer server 13. Thus, the smartphone 11 can periodically send all the data related to temperature measurements compiled from the sensor 10, to the computer server 13. This way, for example, a doctor is able to monitor the temperature of the patient remotely and determine a further treatment, combining the data related to temperature measurements of the patient with other data related to the patient. More precisely, the doctor can, for example, connect to the computer server 13 with a further smartphone or via website, to monitor the temperature or other data related to the patient.

Also, figure 4 depicts the structure of BLE packets. More precisely, a BLE packet 400 comprises a Preamble section 401 (of 1 Byte), an Access section 402 (of 4 Bytes), a Packet Payload 403 (of variable length) and a CRC section 404 (of 3 Bytes). The Packet section 403 embeds a Header section 405 (of 2 Bytes) and a Payload section 406 (of variable length). The Packet section 403 is also known as the Protocol Data Unit (PDU), and depending on the communication mode, it may vary its length and its content. More specifically, two different types of PDUs are possible within BLE: the Advertising Channel PDU (used in advertising mode communications) and the Data Channel PDU (used in connected mode communications).

As seen in figure 4, each PDUs have the same length of Header section (405), which indicate which type of PDU is embedded within the Packet Payload 403.

Furthermore, in Advertising Channel PDU, the Payload section 406 comprises an AdvA section 407 (which corresponds to 6 Bytes comprising the Broadcast Adress), and an AdvData section 408 (which corresponds to a variable length of Bytes comprising the Broadcast Data).

In advertising mode communications, wherein the Advertising Channel PDU is used, it is within the AdvData section 408 that a plurality of Broadcast Data is comprised, which is usually known as Advertisement data types. Such Advertisement data types are a plurality of possible data structures with a specific function each.

Also, in another example, more than one computing devices may be found within the system (for example a first zone receiver and a second zone receiver), and the described method may be performed in parallel by both (for example, if the patient moves around an area covered by both receivers). In such case, both receivers may be connected to the same computer server through a cloud, and in case any duplicate data may be retrieved by both receivers, redundant data may be discarded by the computer server when compiling all the data related to temperature measurements obtained by the sensor worn by the patient.

An example of a practical situation wherein the system may be used may be in a Hospital floor, wherein a vital sign (for example, the body temperature) of a plurality of patients has to be monitored, and in order to do so, each patient may wear a temperature sensor as the ones previously described according to the present disclosure. Furthermore, the Hospital floor may have a plurality of computing devices such as zone receivers, installed in different positions within the floor, which may be configured to receive BLE advertising communication packets from any sensor worn by any patient.

In this example, whenever one or more patients move around the Hospital floor, some zone receivers may receive the BLE advertising packets sent by each sensor, while others may stop receiving such packets (because, for example, the sensor may be move out of reach from the receiver). This way, whenever a receiver loses BLE advertising packets from a sensor, and after a period of time it starts receiving BLE advertising packets from the same sensor (for example, the user has moved to another area of the Hospital floor, and come back to his original area), the zone receiver may retrieve the data related to measurements performed by the sensor while the sensor was out of the receiver's reach. By using several zone receivers, a redundancy of the measurements may be accomplished, thus ensuring no data is lost. However, if the zone receivers are connected to a central server (through a data cloud), the measurements of each sensor may also be available to other receivers, thus not being necessary to a certain receiver to retrieve data related to certain measurements sent while a sensor was out of reach of said receiver. Furthermore, all measurements may be available in real time, and less retrievals of measurements performed while sensors are out of reach may be necessary to be performed by some of the receivers. Therefore, an extra saving of energy may be achieved by using such configuration.

Furthermore, once connectivity between the sensor and receiver device is restored, the data stored in the internal memory can be retrieved from the sensor by the receiver device in an additional way, by retrieving the vital sign data packets in a periodic and automatic way, with configurable frequency (for example, each hour, if a connected mode type of communication can be established). On-demand retrievals performed by a user may also be possible. In this example, when the temperature data retrieval using a connected mode communication is complete, the data is sent to the cloud via an internet connection.

In addition, in this example, the system can also issue alarms when a predefined body temperature limit is exceeded.

Although only a number of examples have been disclosed herein, other alternatives, modifications, uses and/or equivalents thereof are possible. If reference signs related to drawings are placed in parentheses in a claim, they are solely for attempting to increase the intelligibility of the claim and shall not be construed as limiting the scope of the claim.

## Claims

1. A method of remote monitoring of a vital sign of a patient, performed by a computing device (11) comprising a BLE communication module to communicate with a vital sign sensor (10), the method comprising the steps of:
• Receiving a first BLE advertising communication packet comprising a vital sign data packet, from the sensor (10);
• Generating a timestamp for the received first BLE advertising communication packet;
• Performing a first time verification of if the difference between the generated timestamp and a stored timestamp of a previously received BLE advertising communication packet is higher than a predetermined waiting time:
∘ In case of positive result:
▪ Sending a BLE packet from the computing device (11) to the vital sign sensor (10), to establish a BLE communication in connected mode between the vital sign sensor (10) and the computing device (11);
▪ Retrieving, using the BLE communication in connected mode, a plurality of vital sign data packets from the sensor (10), wherein the packets correspond to timestamps with values between the generated timestamp and the stored timestamp.

2. Method according to claim 1, wherein the step of generating a timestamp comprises obtaining a timestamp from the received first BLE advertising communication packet.

3. Method according to claim 1 or 2, wherein the vital sign data packet comprises data corresponding to at least one body temperature measurement performed by the vital sign sensor, and the method further comprises a step of:
• verifying if the body temperature measurement is higher than a predetermined temperature threshold;
and wherein, in case of positive result of the temperature verification, the value of predetermined waiting time is lowered.

4. Method according to any of claims 1 to 3, further comprising the step of:
• verifying if the received signal comprising the first BLE advertising communication packet has a higher power than a predetermined power threshold;
and wherein the step of retrieving a plurality of vital sign data packets from the sensor is performed only in case of positive result of the first power verification.

5. Method according to claim 4, further comprising the steps of:
• receiving a second BLE advertising communication packet after the first received packet; and
• verifying if the received signal comprising the second BLE advertising communication packet has a higher power than a predetermined power threshold;
and wherein the step of retrieving a plurality of vital sign data packets from the sensor is performed only in case of positive result of the first power verification and the second power verification.

6. Method according to claim 5, further comprising the steps of:
• Generating a second timestamp for the second BLE advertising communication packet;
• Performing a second time verification of if the difference between the timestamp of the second BLE advertising communication packet and the timestamp of the first BLE advertising communication packet is lower than a predetermined waiting time;
and wherein the step of retrieving a plurality of vital sign data packets from the sensor is performed only in case of positive result of the first and second power verification and the second time verification.

7. Method according to any of claims 1 to 6, wherein the received first BLE advertising communication packet is embedded in an Eddystone frame.

8. Method according to any of claims 1 to 7, further comprising a step of determining a pattern of availability of the sensor, based on at least a difference between a generated timestamp and a stored timestamp.

9. Computing device (11) for remote monitoring of a vital sign of a patient, comprising
• a BLE communication module to communicate with a vital sign sensor (10);
• a connection to a BLE advertising communication packets repository;
• a controller configured to:
∘ receive a first BLE advertising communication packet comprising a vital sign data packet, from the sensor (10);
∘ generate a timestamp for the received first BLE advertising communication packet;
∘ Perform a first time verification of if the difference between the generated timestamp and a stored timestamp of a previously received BLE advertising communication packet, stored in the repository, is higher than a predetermined waiting time, and:
▪ In case of positive result:
• Send a BLE packet from the computing device (11) to the vital sign sensor (10), to establish a BLE communication in connected mode between the vital sign sensor (10) and the computing device (11);
• Retrieve, using the BLE communication in connected mode, a plurality of vital sign data packets from the sensor (10), wherein the packets correspond to timestamps with values between the generated timestamp and the stored timestamp.

10. Computing device (11) according to claim 9, wherein the device is a smartphone

11. Computing device (11) according to claim 9 or 10, wherein the vital sign sensor is a temperature sensor.

12. A computer program comprising program instructions for causing a computing device to perform a method according to any of claims 1 to 8 of remote monitoring of a vital sign of a patient.

13. A computer program according to claim 12, embodied on a storage medium.

14. A computer program according to any of claims 12 or 13, carried on a carrier signal.

## Patentansprüche

1. Ein Verfahren zur Fernüberwachung eines Vitalzeichens eines Patienten, das von einer Rechenvorrichtung (11) durchgeführt wird, die ein BLE-Kommunikationsmodul umfasst, um mit einem Vitalzeichensensor (10) zu kommunizieren, wobei das Verfahren die folgenden Schritte umfasst:
• Empfangen eines ersten BLE-Werbekommunikationspakets, das ein Vitalzeichendatenpaket umfasst, von dem Sensor (10);
• Erzeugen eines Zeitstempels für das empfangene erste BLE-Werbekommunikationspaket;
• Durchführen einer ersten Zeitüberprüfung davon, ob die Differenz zwischen dem erzeugten Zeitstempel und einem gespeicherten Zeitstempel eines zuvor empfangenen BLE-Werbekommunikationspakets höher als eine vorbestimmte Wartezeit ist:
∘ Im Falle eines positiven Ergebnisses:
▪ Senden eines BLE-Pakets von der Rechenvorrichtung (11) an den Vitalzeichensensor (10), um eine BLE-Kommunikation im verbundenen Modus zwischen dem Vitalzeichensensor (10) und der Rechenvorrichtung (11) einzurichten;
▪ Abrufen einer Vielzahl von Vitalzeichen-Datenpaketen von dem Sensor (10) unter Verwendung der BLE-Kommunikation im verbundenen Modus, wobei die Pakete Zeitstempeln mit Werten zwischen dem erzeugten Zeitstempel und dem gespeicherten Zeitstempel entsprechen.

2. Verfahren nach Anspruch 1, wobei der Schritt des Erzeugens eines Zeitstempels das Erhalten eines Zeitstempels aus dem empfangenen ersten BLE-Werbekommunikationspaket umfasst.

3. Verfahren nach Anspruch 1 oder 2, wobei das Vitalzeichen-Datenpaket Daten umfasst, die mindestens einer Körpertemperaturmessung entsprechen, die von dem Vitalzeichensensor durchgeführt wird, und das Verfahren ferner einen Schritt umfasst bestehend im:
• Verifizieren, ob die Körpertemperaturmessung höher als ein vorbestimmter Temperaturschwellenwert ist;
und wobei im Falle eines positiven Ergebnisses der Temperaturüberprüfung der Wert der vorbestimmten Wartezeit gesenkt wird.

4. Verfahren nach einem der Ansprüche 1 bis 3, ferner umfassend folgenden Schritt:
• verifizieren, ob das empfangene Signal, das das erste BLE-Werbekommunikationspaket umfasst, eine höhere Leistung als einen vorbestimmten Leistungsschwellenwert hat;
und wobei der Schritt des Abrufens einer Vielzahl von Vitalzeichendatenpaketen von dem Sensor nur im Falle eines positiven Ergebnisses der ersten Leistungsüberprüfung durchgeführt wird.

5. Verfahren nach Anspruch 4, ferner umfassend die folgenden Schritte:
• empfangen eines zweiten BLE-Werbekommunikationspakets nach dem ersten empfangenen Paket; und
• verifizieren, ob das empfangene Signal, das das zweite BLE-Werbekommunikationspaket umfasst, eine höhere Leistung als einen vorbestimmten Leistungsschwellenwert hat;
und wobei der Schritt des Abrufens einer Vielzahl von Vitalzeichendatenpaketen von dem Sensor nur im Falle eines positiven Ergebnisses der ersten Leistungsüberprüfung und der zweiten Leistungsüberprüfung durchgeführt wird.

6. Verfahren nach Anspruch 5, ferner umfassend die folgenden Schritte:
• Erzeugen eines zweiten Zeitstempels für das zweite BLE-Werbekommunikationspaket;
• Durchführen einer zweiten Zeitüberprüfung davon, ob die Differenz zwischen dem Zeitstempel des zweiten BLE-Werbekommunikationspakets und dem Zeitstempel des ersten BLE-Werbekommunikationspakets geringer als eine vorbestimmte Wartezeit ist;
und wobei der Schritt des Abrufens einer Vielzahl von Vitalzeichen-Datenpaketen von dem Sensor nur im Falle eines positiven Ergebnisses der ersten und zweiten Leistungsüberprüfung und der zweiten Zeitüberprüfung durchgeführt wird.

7. Verfahren nach einem der Ansprüche 1 bis 6, wobei das empfangene erste BLE-Werbekommunikationspaket in einen Eddystone-Rahmen eingebettet ist.

8. Verfahren nach einem der Ansprüche 1 bis 7, ferner umfassend einen Schritt des Bestimmens eines Musters der Verfügbarkeit des Sensors auf Grundlage von mindestens einer Differenz zwischen einem erzeugten Zeitstempel und einem gespeicherten Zeitstempel.

9. Rechenvorrichtung (11) zur Fernüberwachung eines Vitalzeichens eines Patienten, umfassend:
• ein BLE-Kommunikationsmodul für die Kommunikation mit einem Vitalzeichensensor (10);
• eine Verbindung zu einem BLE-Werbekommunikationspaketen-Repository;
• eine Steuerung, die dazu konfiguriert ist:
∘ Empfangen eines ersten BLE-Werbekommunikationspakets, das ein Vitalzeichendatenpaket umfasst, von dem Sensor (10);
∘ Erzeugen eines Zeitstempels für das empfangene erste BLE-Werbekommunikationspaket;
∘ Durchführen einer ersten Zeitüberprüfung davon, ob die Differenz zwischen dem erzeugten Zeitstempel und einem gespeicherten Zeitstempel eines zuvor empfangenen BLE-Werbekommunikationspakets, das in dem Repository gespeichert ist, höher als eine vorbestimmte Wartezeit ist, und:
▪ Im Falle eines positiven Ergebnisses:
• Senden eines BLE-Pakets von der Rechenvorrichtung (11) an den Vitalzeichensensor (10), um eine BLE-Kommunikation im verbundenen Modus zwischen dem Vitalzeichensensor (10) und der Rechenvorrichtung (11) einzurichten;
• Abrufen einer Vielzahl von Vitalzeichen-Datenpaketen von dem Sensor (10) unter Verwendung der BLE-Kommunikation im verbundenen Modus, wobei die Pakete Zeitstempeln mit Werten zwischen dem erzeugten Zeitstempel und dem gespeicherten Zeitstempel entsprechen.

10. Rechenvorrichtung (11) nach Anspruch 9, wobei die Vorrichtung ein Smartphone ist.

11. Rechenvorrichtung (11) nach Anspruch 9 oder 10, wobei der Vitalzeichensensor ein Temperatursensor ist.

12. Ein Computerprogramm umfassend Programmanweisungen zum Veranlassen einer Rechenvorrichtung, ein Verfahren nach einem der Ansprüche 1 bis 8 zur Fernüberwachung eines Vitalzeichens eines Patienten durchzuführen.

13. Ein Computerprogramm nach Anspruch 12, das in einem Speichermedium enthalten ist.

14. Ein Computerprogramm nach einem der Ansprüche 12 oder 13, das in einem Trägersignal getragen ist.

## Revendications

1. Un procédé de surveillance à distance d'un signe vital d'un patient, mis en oeuvre par un dispositif informatique (11) comprenant un module de communication BLE pour la communication avec un capteur de signes vitaux (10), le procédé comprenant les étapes consistant à :
• Recevoir un premier paquet de communication publicitaire BLE comprenant un paquet de données de signe vital, à partir du capteur (10) ;
• Générer un horodatage pour le premier paquet de communication publicitaire BLE reçu ;
• Effectuer une première vérification temporelle de si la différence entre l'horodatage généré et un horodatage stocké d'un paquet de communication publicitaire BLE précédemment reçu est supérieure à un temps d'attente prédéterminé :
∘ En cas de résultat positif :
▪ Envoyer un paquet BLE du dispositif informatique (11) au capteur de signes vitaux (10), pour établir une communication BLE en mode connecté entre le capteur de signes vitaux (10) et le dispositif informatique (11) ;
▪ Récupérer, à l'aide de la communication BLE en mode connecté, une pluralité de paquets de données de signe vital à partir du capteur (10), dans lequel les paquets correspondent à des horodatages avec des valeurs entre l'horodatage généré et l'horodatage stocké.

2. Procédé selon la revendication 1, dans lequel l'étape de génération d'un horodatage comprend l'obtention d'un horodatage à partir du premier paquet de communication publicitaire BLE reçu.

3. Procédé selon la revendication 1 ou 2, dans lequel le paquet de données de signe vital comprend des données correspondant à au moins une mesure de température du corps effectuée par le capteur de signe vital, et le procédé comprend en outre une étape consistant à :
• vérifier si la mesure de la température du corps est supérieure à un seuil de température prédéterminé ;
et dans lequel, en cas de résultat positif de la vérification de la température, la valeur du temps d'attente prédéterminé est abaissée.

4. Procédé selon l'une quelconque des revendications 1 à 3, comprenant en outre l'étape consistant à :
• vérifier si le signal reçu comprenant le premier paquet de communication publicitaire BLE a une puissance supérieure à un seuil de puissance prédéterminé ;
et dans lequel l'étape de récupération d'une pluralité de paquets de données de signe vital à partir du capteur est effectuée uniquement en cas de résultat positif de la première vérification de puissance.

5. Procédé selon la revendication 4, comprenant en outre les étapes consistant à :
• recevoir un deuxième paquet de communication publicitaire BLE après le premier paquet reçu ; et
• vérifier si le signal reçu comprenant le deuxième paquet de communication publicitaire BLE a une puissance supérieure à un seuil de puissance prédéterminé ;
et dans lequel l'étape de récupération d'une pluralité de paquets de données de signe vital à partir du capteur est effectuée uniquement en cas de résultat positif de la première vérification de puissance et de la seconde vérification de puissance.

6. Procédé selon la revendication 5, comprenant en outre les étapes consistant à :
• Générer un deuxième horodatage pour le deuxième paquet de communication publicitaire BLE ;
• Effectuer une deuxième vérification temporelle de si la différence entre l'horodatage du deuxième paquet de communication publicitaire BLE et l'horodatage du premier paquet de communication publicitaire BLE est inférieure à un temps d'attente prédéterminé ;
et dans lequel l'étape de récupération d'une pluralité de paquets de données de signe vital à partir du capteur est effectuée uniquement en cas de résultat positif de la première et de la deuxième vérification de puissance et de la deuxième vérification temporelle.

7. Procédé selon l'une quelconque des revendications 1 à 6, dans lequel le premier paquet de communication publicitaire BLE reçu est intégré dans une trame d'Eddystone.

8. Procédé selon l'une quelconque des revendications 1 à 7, comprenant en outre une étape consistant à déterminer un modèle de disponibilité du capteur, sur la base d'au moins une différence entre un horodatage généré et un horodatage stocké.

9. Dispositif informatique (11) pour la surveillance à distance d'un signe vital d'un patient, comprenant :
• un module de communication BLE pour la communication avec un capteur de signes vitaux (10) ;
• une connexion à un référentiel de paquets de communication publicitaire BLE ;
• un contrôleur configuré pour :
∘ Recevoir un premier paquet de communication publicitaire BLE comprenant un paquet de données de signe vital, à partir du capteur (10) ;
∘ Générer un horodatage pour le premier paquet de communication publicitaire BLE reçu ;
∘ Effectuer une première vérification temporelle de si la différence entre l'horodatage généré et un horodatage stocké d'un paquet de communication publicitaire BLE précédemment reçu, stocké dans le référentiel, est supérieure à un temps d'attente prédéterminé, et :
▪ En cas de résultat positif :
• Envoyer un paquet BLE du dispositif informatique (11) au capteur de signes vitaux (10), pour établir une communication BLE en mode connecté entre le capteur de signes vitaux (10) et le dispositif informatique (11) ;
• Récupérer, à l'aide de la communication BLE en mode connecté, une pluralité de paquets de données de signe vital à partir du capteur (10), dans lequel les paquets correspondent à des horodatages avec des valeurs entre l'horodatage généré et l'horodatage stocké.

10. Dispositif informatique (11) selon la revendication 9, dans lequel le dispositif est un smartphone.

11. Dispositif informatique (11) selon la revendication 9 ou 10, dans lequel le capteur de signes vitaux est un capteur de température.

12. Un programme informatique comprenant des instructions de programme pour amener un dispositif informatique à exécuter un procédé selon l'une quelconque des revendications 1 à 8 de surveillance à distance d'un signe vital d'un patient.

13. Un programme informatique selon la revendication 12, incorporé dans un support de stockage.

14. Un programme informatique selon l'une quelconque des revendications 12 ou 13, porté dans un signal porteur.
